# EUROPÄISCHE PATENTSCHRIFT

(11) **EP 1 856 070 B1**
(45) Veröffentlichungstag und Bekanntmachung des Hinweises auf die Patenterteilung: **27.07.2011**
(21) Anmeldenummer: 06708325.3
(22) Anmeldetag: 16.02.2006
(51) Int. Cl.: C07D 265/18, A61K 31/536

(54) **NEUE ARZNEIMITTEL ZUR BEHANDLUNG VON ATEMWEGSERKRANKUNGEN**
NOVEL DRUGS FOR TREATING RESPIRATORY DISEASES
NOUVEAUX MEDICAMENTS POUR TRAITER DES MALADIES DES VOIES RESPIRATOIRES

(30) Priorität: 24.02.2005 DE 102005008921
(43) Veröffentlichungstag der Anmeldung: 21.11.2007
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: KONETZKI, Ingo, 88447 Warthausen (DE); BOUYSSOU, Thierry, 88447 Warthausen (DE); LUSTENBERGER, Philipp, CH-4056 Basel (CH); SCHNAPP, Andreas, 88400 Biberach (DE)
(74) Vertreter: Hammann, Heinz
(86) Internationale Anmeldenummer: PCT/EP2006/060033
(87) Internationale Veröffentlichungsnummer: WO 2006/089859

(56) Entgegenhaltungen:
- EP-A- 0 043 940
- WO-A-2005/070908

## Beschreibung

Die vorliegende Erfindung betrifft die Verwendung der Verbindungen der allgemeinen Formel **1** 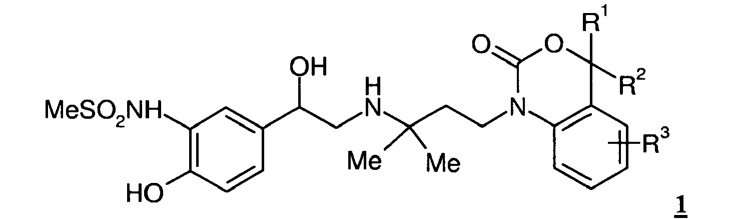 worin die Reste R¹, R² und R³ die in den Ansprüchen und in der Beschreibung genannten Bedeutungen haben können, zur Herstellung eines Arzneimittels zur Behandlung von Atemwegserkrankungen, sowie neue Verbindungen der Formel **1**, Verfahren zu deren Herstellung, und diese enthaltende pharmazeutische Formulierungen.

### Hintergrund der Erfindung

Betamimetika (β-adrenerge Substanzen) sind aus dem Stand der Technik bekannt. Beispielsweise sei diesbezüglich auf die Offenbarung der US 4,341,778 oder der EP 43940 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.
Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die einerseits bei der Therapie von Atemwegserkrankungen einen therapeutischen Nutzen entfalten und darüber hinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von Asthma einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### Detaillierte Beschreibung der Erfindung

Überraschenderweise wurde gefunden, dass die vorstehend genannten Aufgaben durch Verbindungen der allgemeinen Formel **1** gelöst werden.

Dementsprechend betrifft die vorliegende Erfindung die Verwendung einer oder mehrerer, bevorzugt einer Verbindung der allgemeinen Formel **1** 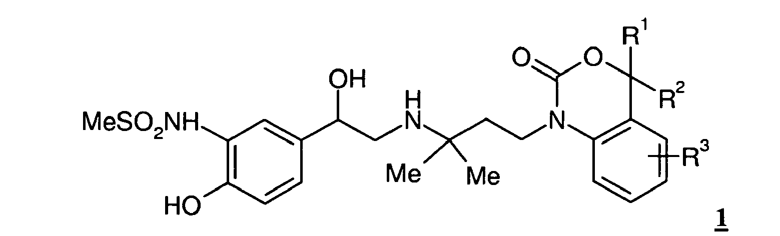 worin
- R¹ und R²: gleich oder verschieden, Wasserstoff, Halogen, -C₁-C₄-Alkyl oder gemeinsam -C₁-C₆-Alkylen und
- R³: Wasserstoff, Halogen, OH, C₁-C₄-Alkyl oder -O-C₁-C₄-Akyl;
bedeuten, zur Herstellung eines Arzneimittels zur Behandlung von

Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die vorstehend genannte Verwendung von Verbindungen der allgemeinen Formel **1**, worin
- R¹ und R²: gleich oder verschieden, Wasserstoff, Fluor, Chlor, Methyl, Ethyl, Propyl, Butyl oder gemeinsam -CH₂-CH₂-, -CH₂-CH₂-CH₂-, -CH₂-CH₂-CH₂-CH₂- oder -CH₂-CH₂-CH₂-CH₂-CH₂-;
- R³: Wasserstoff, Fluor, Chlor, OH, Methyl, Ethyl, Methoxy, oder Ethoxy
bedeuten.

Bevorzugt ist ferner die vorstehend genannte Verwendung von Verbindungen der allgemeinen Formel **1**, worin
- R¹ und R²: gleich oder verschieden, Wasserstoff, Methyl, Ethyl, Propyl oder gemeinsam -CH₂-CH₂-, -CH₂-CH₂-CH₂-, -CH₂-CH₂-CH₂-CH₂- oder -CH₂-CH₂-CH₂-CH₂-CH₂-;
- R³: Wasserstoff, Fluor, OH, Methyl oder Methoxy bedeuten.

Bevorzugt ist die Verwendung von Verbindungen der allgemeinen Formel **1** zur Herstellung eines Arzneimittels zur Behandlung von Obstruktiven Lungenerkrankungen die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale oder COPD erfindungsgemäß besonders bevorzugt ist.

Bevorzugt ist ferner die Verwendung von Verbindungen der allgemeinen Formel **1** zur Herstellung eines Arzneimittels zur Behandlung von Lungenemphysemen, die ihren Ursprung haben in COPD oder α1-Proteinase-Inhibitor-Mangel.

Bevorzugt ist ferner die Verwendung von Verbindungen der allgemeinen Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der allgemeinen Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der allgemeinen Formel **1** zur Herstellung eines Arzneimittels zur Behandlung von Zystischer Fibrose bzw. Mukoviszidose.

Bevorzugt ist ferner die Verwendung von Verbindungen der allgemeinen Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der allgemeinen Formel **1** zur Herstellung eines Arzneimittels zur Behandlung von Bronchiektasen.

Bevorzugt ist ferner die Verwendung von Verbindungen der allgemeinen Formel **1** zur Herstellung eines Arzneimittels zur Behandlung von ARDS (adult respiratory distress syndrom).

Bevorzugt ist ferner die Verwendung von Verbindungen der allgemeinen Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Besonders bevorzugt ist die vorstehend genannte Verwendung von Verbindungen der Formel **1**, die ausgewählt sind aus der Gruppe bestehend aus
- N-(5-{2-[1,1-Dimethyl-3-(4-methyl-2-oxo-4H-benzo[d][1,3]oxazin-1-yl)-propylamino]-1-hydroxy-ethyl}-2-hydroxy-phenyl)-methansulfonamid;
- N-(5-{2-[1,1-Dimethyl-3-(2-oxo-4H-benzo[d][1,3]oxazin-1-yl)-propylamino]-1-hydroxy-ethyl }-2-hydroxy-phenyl)-methansulfonamid;
- N-(5-{2-[3-(4-Ethyl-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Dimethyl-2-oxo-4H-benzo[d] [1,3]oxazin-1-yl)-1,1-dimethyl-propylamino]-1-hydroxy-ethyl}-2-hydroxy-phenyl)-methansulfonamid;
- N-(2-Hydroxy-5-{1-hydroxy-2-[3-(6-hydroxy-4,4-dimethyl-2-oxo-4H-benzo[d][1,3]oxazin-l-yl)-1,1-dimethyl-propylamino]-ethyl}-phenyl)-methansulfonamid;
- N-(2-Hydroxy-5-{1-hydroxy-2-[3-(6-methoxy-4,4-dimethyl-2-oxo-4H-benzo[d][1,3]oxazin-1-yl)-1,1-dimethyl-propylamino]-ethyl}-phenyl)-methansulfonamid.

Die Verbindungen der Formel **1** sind so wie die vorstehend explizit genannten Verbindungen teilweise aus dem Stand der Technik bekannt. Hierzu sei insbesondere auf die Offenbarung der Dokumente EP 43940 und US 4341778 verwiesen.

Die vorliegende Erfindung betrifft darüber hinaus neue Verbindungen der Formel **1** als solche. Dies sind insbesondere solche Verbindungen der Formel **1**, in denen R¹ und R² gleich oder verschieden, bevorzugt gleich Ethyl oder Propyl, oder gemeinsam -CH₂-CH₂-, -CH₂-CH₂-CH₂-, -CH₂-CH₂-CH₂-CH₂- oder - CH₂-CH₂-CH₂-CH₂-CH₂- bedeuten und in denen
- R³: Wasserstoff, Fluor, Chlor, OH, Methyl, Ethyl, Methoxy oder Ethoxy
sein kann.

Besonders bevorzugt sind die Verbindungen der Formel **1**, in denen
- R¹ und R²: gleich oder verschieden, bevorzugt gleich, Ethyl oder Propyl, oder gemeinsam -CH₂-CH₂-, -CH₂-CH₂-CH₂-CH₂- oder -CH₂-CH₂-CH₂-CH₂-CH₂- bedeuten und in denen
- R³: Wasserstoff, Fluor, OH, Methyl oder Methoxy, bevorzugt Wasserstoff
sein kann.

Besonders bevorzugt sind die Verbindungen der Formel **1**, in denen
- R¹ und R²: gleich Ethyl oder Propyl bedeuten und in denen
- R³: Wasserstoff, Fluor, OH, Methyl oder Methoxy, bevorzugt Wasserstoff
sein kann.

Besonders bevorzugt sind die Verbindungen der Formel **1**, in denen
- R¹ und R²: gemeinsam -CH₂-CH₂-, -CH₂-CH₂-CH₂-CH₂- oder -CH₂-CH₂-CH₂-CH₂-CH₂- bedeuten und in denen
- R³: Wasserstoff, Fluor, OH, Methyl oder Methoxy, bevorzugt Wasserstoff
sein kann.

Besonders bevorzugt sind die Verbindungen der Formel **1**, in denen R³ Wasserstoff bedeutet und R¹ und R² die vorstehend genannten Bedeutungen haben können.

Besonders bevorzugt sind die Verbindungen der Formel **1**, in denen R³ Fluor bedeutet und R¹ und R² die vorstehend genannten Bedeutungen haben können.

Besonders bevorzugt sind die Verbindungen der Formel **1**, in denen R¹ und R² beide Ethyl oder Propyl bedeuten und in denen R³ die vorstehend genannten Bedeutungen haben kann.

Besonders bevorzugt sind diejenigen Verbindungen der Formel **1**, die ausgewählt sind aus der Gruppe bestehend aus
- N-(5-{2-[1,1-Dimethyl-3-(2-oxo-4,4-dipropyl-4H-benzo[d][1,3]oxazin-1-yl)-propylamino]-1-hydroxy-ethyl }-2-hydroxy-phenyl)-methansulfonamid;
- N-[5-(2-{1,1-Dimethyl-3-[spiro(cyclohexan-1,4'-2H-3',1'-benzoxazin)-2'-oxo-1-yl]-propylamino}-1-hydroxy-ethyl)-2-hydroxy-phenyl]-methansulfonamid;
- N-[5-(2-{1,1-Dimethyl-3-[spiro(cydopropyl-1,4'-2H-3',1'-benzoxazin)-2'-oxo-1-yl]-propylamino}-1-hydroxy-ethyl)-2-hydroxy-phenyl]-methansulfonamid;
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-7-fluoro-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-8-methoxy-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid.

Ein weiterer Aspekt der vorliegenden Erfindung betrifft die vorstehend genannten neuen Verbindungen der allgemeinen Formel **1** als Arzneimittel. Ferner betrifft die vorliegende Erfindung die Verwendung vorstehend genannter neuer Verbindungen der Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Ein weiterer Aspekt der vorliegenden Erfindung betrifft die vorstehend genannte Verwendung der Verbindungen der Formel **1** in Form der einzelnen optischen Isomeren, Mischungen der einzelnen Enantiomeren, Diastereomeren oder Racemate. Besonders bevorzugt ist hierbei die vorstehend genannte Verwendung der Verbindungen der Formel **1** in Form der enantiomeren- beziehungsweise diastereomerenreinen Verbindungen, wobei die Verwendung der R-Enantiomere der Verbindungen der Formel ***R*-1** 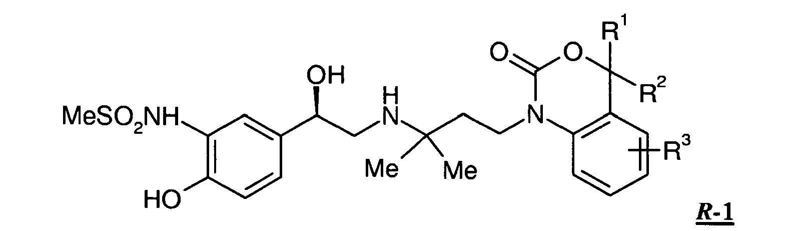 in denen die Reste R¹, R² und R³ die vorstehend genannten Bedeutungen haben können, erfindungsgemäß von herausragender Bedeutung ist.

Ein weiterer Aspekt der vorliegenden Erfindung betrifft die vorstehend genannten neuen Verbindungen der Formel **1** in Form der einzelnen optischen Isomeren, Mischungen der einzelnen Enantiomeren, Diastereomeren oder Racemate. Besonders bevorzugt sind hierbei die vorstehend genannten neuen Verbindungen der Formel **1** in Form der enantiomerenbeziehungsweise diastereomerenreinen Verbindungen, wobei die R-Enantiomere der Formel ***R*-1** erfindungsgemäß von herausragender Bedeutung ist.

Ein weiterer Aspekt der vorliegenden Erfindung betrifft die vorstehend genannte Verwendung der Verbindungen der Formel **1** in Form der freien Basen oder in Form der Säureadditionssalze mit pharmakologisch unbedenklichen Säuren, sowie gegebenenfalls in Form der Solvate und/oder Hydrate.

Ein weiterer Aspekt der vorliegenden Erfindung betrifft die vorstehend genannten neuen Verbindungen der Formel **1** in Form der freien Basen oder in Form der Säureadditionssalze mit pharmakologisch unbedenklichen Säuren, sowie gegebenenfalls in Form der Solvate und/oder Hydrate.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl n-Propyl und iso-Propyl, Butyl umfasst iso-Butyl, sec. Butyl und tert.-Butyl etc.

Als Alkylengruppen werden, soweit nicht anders angegeben, verzweigte und unverzweigte zweibindige Alkylbrücken mit 1 bis 6 Kohlenstoffatomen bezeichnet. Beispielsweise werden genannt: Methylen, Ethylen, n-Propylen oder n-Butylen.

Als Alkyloxygruppen (oder auch -O-Alkylgruppen) werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfasst beispielsweise Propyloxy n-Propyloxy und iso-Propyloxy, Butyloxy umfasst iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Die Herstellung der erfindungsgemäßen Verbindungen kann gemäß oder in Analogie zu im Stand der Technik bereits bekannten Vorgehensweisen erfolgen. Geeignete Herstellverfahren sind beispielsweise aus der EP43940 oder der US 4341778 bekannt, auf die an dieser Stelle vollinhaltlich Bezug genommen wird.

Die nachstehend beschriebenen Beispiele dienen der weitergehenden Illustration von aus dem Stand der Technik bekannten Verbindungen, die entsprechend der vorliegenden Erfindung überraschenderweise zur Therapie der vorstehend genannten Atemwegserkrankungen Verwendung finden können.

### Beispiel 1: N-(5-{2-[1,1-Dimethyl-3-(4-methyl-2-oxo-4H-benzo[d][1,3]oxazin-1-yl)-propylamino]-1-hydroxy-ethyl}-2-hydroxy-phenyl)-methansulfonamid

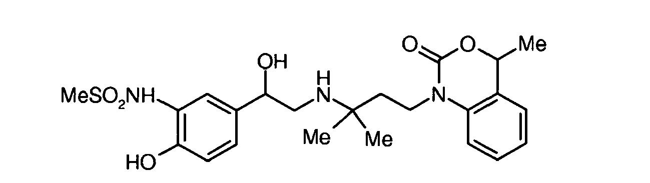

Die Verbindung ist aus der EP 43940 bekannt. Die einzelnen Diastereomere dieses Ausführungsbeispiels können nach gängigen, im Stand der Technik bekannten Methoden erhalten werden.

### Beispiel 2: N-(5-{2-[1,1-Dimethyl-3-(2-oxo-4H-benzo[d][1,3]oxazin-1-yl)-propylamino]-1-hydroxy-ethyl}-2-hydroxy-phenyl)-methansulfonamid

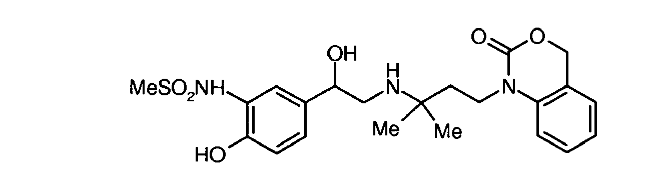

Die Verbindung ist aus der EP 43940 bekannt. Die (R)- und (S)-Enantiomere dieses Ausführungsbeispiels können nach gängigen, im Stand der Technik bekannten Methoden erhalten werden.

### Beispiel 3: N-(5-{2-[3-(4-Ethyl-2-oxo-4H-benzo[d][1,3]oxazin-1-yl)-1,1-dimethyl-propylamino]-1-hydroxy-ethyl}-2-hydroxy-phenyl)-methansulfonamid

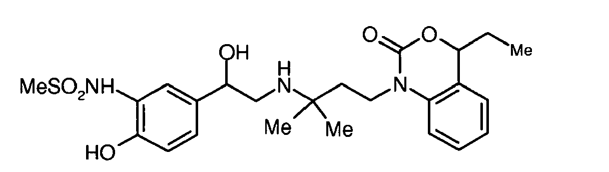

Die Verbindung ist aus der EP 43940 bekannt. Die einzelnen Diastereomere dieses Ausführungsbeispiels können nach gängigen, im Stand der Technik bekannten Methoden erhalten werden.

### Beispiel 4: N-(5-{2-[3-(4,4-Dimethyl-2-oxo-4H-benzo[d][1,3]oxazin-1-yl)-1,1-dimethyl-propylamino]-1-hydroxy-ethyl}-2-hydroxy-phenyl)-methansulfonamid

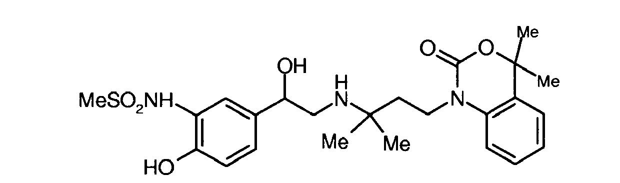

Die Verbindung ist aus der EP 43940 bekannt. Die (R)- und (S)-Enantiomere dieses Ausführungsbeispiels können nach gängigen, im Stand der Technik bekannten Methoden erhalten werden.

### Beispiel 5: N-(2-Hydroxy-5-{1-hydroxy-2-{3-(6-hydroxy-4,4-dimethyl-2-oxo-4H-benzo[d][1,3]oxazin-1-yl)-1,1-dimethyl-propylaminol-ethyl}-phenyl)-methansulfonamid

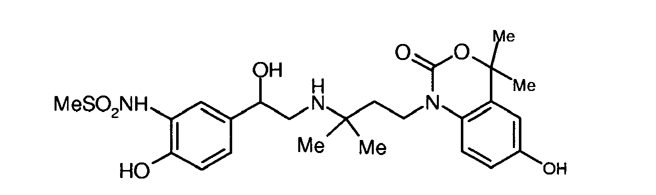

Die Verbindung ist aus der EP 43940 bekannt. Die (R)- und (S)-Enantiomere dieses Ausführungsbeispiels können nach gängigen, im Stand der Technik bekannten Methoden erhalten werden.

### Beispiel 6: N-(2-Hydroxy-5-{1-hydroxy-2-[3-(6-methoxy-4,4-dimethyl-2-oxo-4H-benzo[d][1,3]oxazin-1-yl)-1,1-dimethyl-propylamino]-ethyl}-phenyl)-methansulfonamid

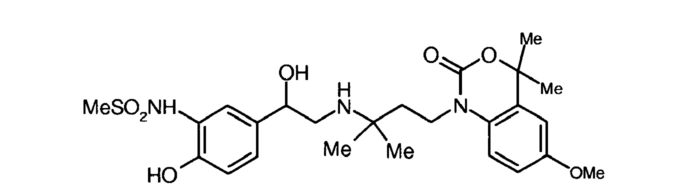

Die Verbindung ist aus der EP 43940 bekannt. Die (R)- und (S)-Enantiomere dieses Ausführungsbeispiels können nach gängigen, im Stand der Technik bekannten Methoden erhalten werden.

Die nachstehend beschriebenen Synthesebeispiele dienen der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

HPLC-Methode (Methode A): Symmetry C18 (Waters): 3.5 µm; 4.6 x 150 mm; Säulentemperatur: 20°C; Gradient: Acetonitril/Phosphat-Puffer (pH 7) 20:80 ? 80:20 in 30 Minuten; Fluss: 1.0 mL / min; Detektion bei 220 und 254 nm.

### Synthese der Zwischenprodukte 1- 8

### Zwischenprodukt 1: 1-(3-Amino-3-methyl-butyl)-4,4-dipropyl-1,4-dihydro-benzo[d][1,3]oxazin-2-on

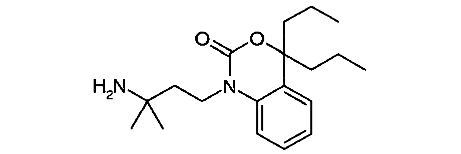

### a) 4-(2-Amino-phenyl)-heptan-4-ol

Zu einer Lösung von 7.00 mL (54.0 mmol) Anthranilsäuremethylester in abs. THF (70 mL) werden bei 0°C innerhalb von 30 Minuten 90 mL (180.0 mmol) Propylmagnesiumchlorid (2 M in Ether) zugetropft. Das Gemisch wird eine Stunde bei Raumtemperatur gerührt und dann mit 100 mL 3 molarer wässriger Ammoniumchlorid-Lösung und Ethylacetat versetzt. Die Phasen werden getrennt und die wässrige Phase wird erschöpfend mit Ethylacetat extrahiert. Die vereinigten organischen Phasen werden mit Kaliumhydrogencarbonat-Lösung und gesättigter Natriumchlorid-Lösung gewaschen und mit Natriumsulfat getrocknet. Das Rohprodukt wird ohne weitere Reinigung in dem nachfolgenden Reaktionsschritt eingesetzt. Ausbeute: 6.70 g (60%).

### b) {3-[2-(1-Hydroxy-1-propyl-butyl)-phenylamino]-1,1-dimethyl-propyl}-carbaminsäure-tert-butylester

Zu einer Lösung von 3.10 g (14.05 mmol) 4-(2-Amino-phenyl)-heptan-4-ol und 3.60 g (17.88 mmol) (1,1-Dimethyl-3-oxo-propyl)-carbaminsäure-tert-butylester in Methanol (40 mL) und Essigsäure (6 mL) werden 1.40 g (22.27 mmol) Natriumcyanoborhydrid zugegeben. Das Gemisch wird 16 Stunden bei Raumtemperatur gerührt, mit Ethylacetat verdünnt, mit 0.5 molarer Kaliumhydrogensulfat-Lösung und gesättigter Natriumchlorid-Lösung gewaschen, mit Natriumsulfat getrocknet und im Vakuum eingeengt. Das Rohprodukt wird ohne weitere Reinigung in dem nachfolgenden Reaktionsschritt eingesetzt. Ausbeute: 6.00 g (quantitative Ausbeute).

### c) [1,1-Dimethyl-3-(2-oxo-4,4-dipropyl-4H-benzo[d][1,3]oxazin-1-yl)-propyl]-carbaminsäure-tert-butylester

Zu einer Lösung von 6.00 g (15.28 mmol) {3-[2-(1-Hydroxy-1-propyl-butyl)-phenylamino]-1,1-dimethyl-propyl}-carbaminsäure-tert-butylester und 5.32 mL (38.21 mmol) Triethylamin in abs. THF (80 mL) werden bei 0°C 8.85 mL (16.81 mmol) Phosgen-Lösung (20 gew.% in Toluol) langsam zugetropft. Das Gemisch wird 2 Stunden bei Raumtemperatur gerührt, mit Ethylacetat verdünnt, mit Eis versetzt und mit gesättigter wässriger Ammoniak-Lösung basisch gestellt. Die wässrige Phase wird mit Ethylacetat erschöpfend extrahiert und die vereinigten organischen Phasen werden mit gesättigter Natriumchlorid-Lösung gewaschen, mit Natriumsulfat getrocknet und im Vakuum eingeengt. Nach Säulenchromatographie (Kieselgel, Cyclohexan/Ethylacetat = 6:1) wird das Produkt als gelbes Öl erhalten. Ausbeute: 4.57 g (71%).

### d) 1-(3-Amino-3-methyl-butyl)-4,4-dipropyl-1,4-dihydro-benzo[d][1,3]oxazin-2-on

Eine Lösung von 4.20 g (10.03 mmol) [1,1-Dimethyl-3-(2-oxo-4,4-dipropyl-4*H-*benzo[*d*][1,3]oxazin-1-yl)-propyl]-carbaminsäure-tert-butylester in 35 mL Ameisensäure wird 24 Stunden bei Raumtemperatur gerührt und anschließend auf Eis gegossen. Die wässrige Phase wird mit gesättigter wässriger Ammoniak-Lösung basisch gestellt und mit Ethylacetat erschöpfend extrahiert. Die vereinigten organischen Extrakte werden mit Natriumchlorid-Lösung gewaschen, mit Natriumsulfat getrocknet und im Vakuum eingeengt. Der Rückstand wird in Ethylacetat (50 mL) aufgenommen und mit 4 mL Salzsäure in Ethylacetat (gesättigt) versetzt. Die Lösung wird eingedampft und zweimal mit wenig Ethanol versetzt und im Vakuum eingeengt. Verreiben des Rückstands mit Diisopropylether ergibt das Produkt als hygroskopisches Hydrochloridsalz. Ausbeute: 2.60 g (73%).

### Zwischenprodukt 2: 1-(3-Amino-3-methyl-butyl)-4,4-diethyl-7-fluor-1,4-dihydro-benzo[d][1,3]oxazin-2-on

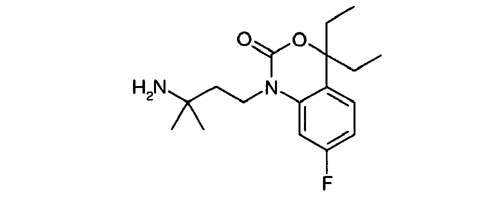

### a) 3-(2-Amino-4-fluor-phenyl)-pentan-3-ol

Das Produkt wird analog Zwischenprodukt 1a durch Reaktion von 2-Amino-4-fluor-benzoesäuremethylester und Ethylmagnesiumbromid in Dichlormethan bei -78°C unter Erwärmung auf Raumtemperatur erhalten. Ausbeute: 4.1 g (99%).

### b) {3-[2-(1-Ethyl-1-hydroxy-propyl)-5-fluor-phenylamino]1,1-dimethyl-propyl}-carbaminsäure-tert-butylester

Das Produkt wird analog Zwischenprodukt 1b ausgehend von 3-(2-Amino-4-fluor-phenyl)-pentan-3-ol und (1,1-Dimethyl-3-oxo-propyl)-carbaminsäure-tert-butylester erhalten. Das Rohprodukt wird mittels Säulenchromatographie (Kieselgel, Dichlormethan/Methanol = 100:0 ? 98:2) gereinigt. Ausbeute: 7.70 g (99%).

### c) [3-(4,4-Diethyl-7-fluor-2-oxo-4H-benzo[d][1,3]oxazin-1-yl)-1,1-dimethyl-propyl]-carbaminsäure-tert-butylester

Das Produkt wird analog Zwischenprodukt 1c ausgehend von {3-[2-(1-Ethyl-1-hydroxypropyl)-5-fluor-phenylamino]-1,1-dimethyl-propyl}-carbaminsäure-tert-butylester erhalten. Ausbeute: 4.20 g (51%).

### d) 1-(3-Amino-3-methyl-butyl)-4,4-diethyl-7-fluor-1,4-dihydro-benzo[d][1,3]oxazin-2-on

Das Produkt wird analog Zwischenprodukt 1d ausgehend von [3-(4,4-Diethyl-7-fluor-2-oxo-4H-benzo[d][1,3]oxazin-1-yl)-1,1-dimethyl-propyl]-carbaminsäure-tert-butylester als freie Base hergestellt. Ausbeute: 2.90 g (96%); ESI-MS: [M+H]⁺ = 309.

### Zwischenprodukt 3: 1-(3-Amino-3-methyl-butyl)-spiro(cyclopropyl-1,4'-2H-3',1'-benzoxazin)-2'-on

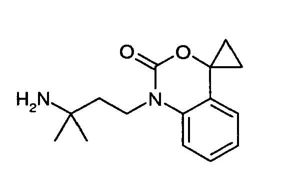

### a) 1-(2-Dibenzylamino-phenyl)-cyclopropanol

Zu einer Lösung von 18.5 g (55.8 mmol) 2-Dibenzylamino-benzoesäuremethylester in 150 mL THF werden bei Raumtemperatur 2.45 mL (8.4 mmol) Titantetraisopropylat langsam zugetropft. Nach einer Stunde rühren werden 40.9 mL (122.7 mmol) Ethylmagnesiumbromid (3 M in Diethylether) zugegeben. Man läßt eine Stunde rühren, gibt weitere 4 mL 3 molare Ethylmagnesiumbromid-Lösung zu und rührt 2 Stunden. Die Reaktionsmischung wird mit gesättigter Ammoniumchlorid-Lösung versetzt und mit Ethylacetat extrahiert. Die wässrige Phase wird mit 1 molarer Salzsäure versetzt bis eine klare Lösung vorliegt und mit Ethylacetat extrahiert. Die vereinigten organischen Phasen werden mit Natriumhydrogencarbonat-Lösung und Natriumchlorid-Lösung gewaschen, mit Natriumsulfat getrocknet und eingeengt. Der Rückstand wird chromatographisch gereinigt (Hexan/Ethylacetat = 20:1). Gelbes Öl. Ausbeute: 10.0 g (54%).

### b) 1-(2-Amino-phenyl)-cyclopropanol

9.90 g (30.1 mmol) 1-(2-Dibenzylamino-phenyl)-cyclopropanol werden in 70 mL Methanol gelöst und in Gegenwart von 1 g Palladium auf Kohle (10%ig) bei 3 bar Wasserstoffdruck hydriert. Der Katalysator wird abgesaugt, das Filtrat eingeengt und der Rückstand chromatographisch gereinigt (Kieselgel; Cyclohexan/Ethylacetat = 5:1). Weißer Feststoff. Ausbeute: 1.80 g (40%).

### c) {3-12-(1-Hydroxy-cyclopropyl)-phenylamino]-1,1-dimethyl-propyl}-carbaminsäure tert-butylester

Herstellung in Analogie zu dem für das Zwischenprodukt 1b beschriebenen Verfahren aus 1.77 g (11.86 mmol) 1-(2-Amino-phenyl)-cyclopropanol und 3.15 g (15.66 mmol) (1,1-Dimethyl-3-oxo-propyl)-carbaminsäure-tert-butylester. Das erhaltene Rohprodukt wird mittels Säulenchromatographie (Kieselgel, Cyclohexan/Ethylacetat 4:1) gereinigt. Gelbes Öl. Ausbeute: 2.60 g.

### d) {1,1-Dimethyl-3-[spiro(cycloproyl-1,4'-2H-3',1'-benzoxazin)-2'-oxo-1-yl]-propyl}-carbaminsäure-tert-butylester

Das Produkt wird analog Zwischenprodukt 1c ausgehend von 2.60 g (7.74 mmol) {3-[2-(1-Hydroxy-cyclopropyl)-phenylamino]-1,1-dimethyl-propyl}-carbaminsäure tert-butylester erhalten. Abweichend wird auf eine säulenchromatographische Reinigung verzichtet. Gelbes Öl. Ausbeute: 2.60 g.

### e) 1-(3-Amino-3-methyl-butyl)-spiro(cyclopropyl-1,4'-2H-3',1'-benzoxazin)-2'-on

Erhalten in Analogie zu dem für die Zwischenstufe 1d beschriebenen Verfahren aus der Umsetzung von 3.10 g (8.60 mmol){1,1-Dimethyl-3-[spiro(cycloproyl-1,4'-2H-3',1'-benzoxazin)-2'-oxo-1-yl]-propyl}-carbaminsäure-tert-butylester und 30 mL Ameisensäure. Gelbes Öl. Ausbeute: 2.10 g (94%).

### Zwischenprodukt 4: 1-(3-Amino-3-methyl-butyl)-4,4-diethyl-1,4-dihydro-benzo[d][1,3]oxazin-2-on

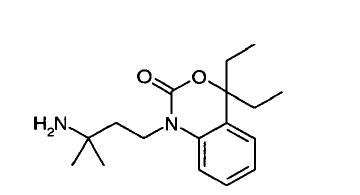

### a) 3-(2-Amino-phenyl)-pentan-3-ol

Zu einer Lösung von 7.77 mL (60 mmol) 2-Amino-methylbenzoesäure in 130 mL THF werden bei -40°C 100 mL einer 3 molaren Ethylmagnesiumbromid-Lösung in Diethylether getropft. Es wird über Nacht unter Erwärmung auf Raumtemperatur gerührt, mit gesättigter Ammoniumchlorid-Lösung versetzt, mit 1 molarer Salzsäure angesäuert und mit Ethylacetat extrahiert. Die vereinigten organischen Phasen werden mit Wasser extrahiert, mit Natriumsulfat getrocknet und eingeengt. Dunkelrotes Öl, das auskristallisiert und direkt weiter umgesetzt wird. Ausbeute: 10.9 g; Massenspektroskopie: [M+H]⁺ = 180.

### b) {3-[2-(1-Ethyl-1-hydroxy-propyl)-phenylamino]-1,1-dimethyl-propyl}-carbaminsäure-tert-butylester

Zu 5.70 g (31.8 mmol) 3-(2-Amino-phenyl)-pentan-3-ol und 2.63 mL (47.7 mmol) Essigsäure in 18 mL Methanol werden bei Raumtemperatur 3.16 g (47.7 mmol) Natriumcyanoborhydrid gegeben. Anschließend wird eine Lösung von 7.04 g (35 mmol) (1,1-Dimethyl-3-oxo-propyl)-carbaminsäure-tert-butylester in 18 mL Methanol langsam zugetropft. Nach beendeter Zugabe wird vier Stunden gerührt, mit 1 molarer Salzsäure versetzt (Gasentwicklung) und dann mit wässriger Ammoniak-Lösung basisch gestellt. Es wird mit Ethylacetat extrahiert und die vereinigten organischen Phasen werden mit Natriumchlorid-Lösung gewaschen, mit Natriumsulfat getrocknet und vom Lösungsmittel befreit. Der Rückstand wird mittels Säulenchromatographie gereinigt (Kieselgel, Dichlormethan/Methanol-Gradient mit 0.1 % Ammoniak). Gelbes Öl. Ausbeute: 4.25 g (37%); Massenspektroskopie: [M+H]⁺ = 365.

### c) [3-(4,4-Diethyl-2-oxo-4H-benzo[d][1,3]oxazin-1-yl)-1,1-dimethyl-propyl]-carbaminsäure-tert-butylester

Zu einer Lösung von 3.50 g (9.6 mmol) {3-[2-(1-Ethyl-1-hydroxy-propyl)-phenylamino]-1,1-dimethyl-propyl}-carbaminsäure-tert-butylester und 3.37 mL (24 mmol) Triethylamin in 35 mL THF werden bei 0 bis 5°C 2.91 g (9.6 mmol) Triphosgen gegeben. Man läßt über Nacht bei Raumtemperatur rühren und saugt den entstandenen Niederschlag ab. Das Filtrat wird eingeengt und das zurückbleibende Öl direkt weiter umgesetzt. Ausbeute: 3.33 g; Massenspektroskopie: [M+H]⁺ = 391.

### d) 1-(3-Amino-3-methyl-butyl)-4,4-diethyl-1,4-dihydro-benzo[d][1,3]oxazin-2-on

Zu einer Lösung von 3.20 g [3-(4,4-Diethyl-2-oxo-4H-benzo[d][1,3]oxazin-1-yl)-1,1-dimethyl-propyl]-carbaminsäure-tert-butylester (ca. 75%ig) in 25 mL Dichlormethan werden unter Kühlung mit dem Eisbad 25 mL Trifluoressigsäure getropft. Man läßt 2 Stunden bei Raumtemperatur rühren, destilliert die Lösungsmittel ab und entfernt Säurereste durch wiederholte Kodestillation mit Toluol. Zur Freisetzung der freien Base wird der Rückstand mit 1 molarer Natronlauge versetzt und mit Ethylacetat extrahiert. Die organischen Phasen werden mit Natriumsulfat getrocknet und eingeengt. Die freie Base wird in 8 mL Methanol gelöst und mit etherischen Salzsäure versetzt. Es wird über Nacht gerührt und der entstandene Niederschlag wird abgesaugt und mit Diethylether gewaschen. Ausbeute: 2.15 g (Hydrochlorid); Massenspektroskopie: [M+H]⁺ = 291.

### Zwischenprodukt 5: 1-(3-Amino-3-methyl-butyll)-spiro(cyclohexan-1,4'-2H-3',1'-benzoxazin)-2'-on

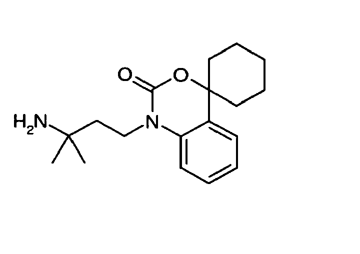

### a) 1-(2-Nitro-phenyl)-cyclohexanol

Zu einer Lösung von 20.0 g (80.32 mmol) 2-Nitro-iodbenzol in 150 mL THF werden bei - 50 °C unter Stickstoff 40.16 mL (80.32 mmol) Phenylmagnesiumchlorid (2 M in THF) zugetropft. Nach 15 Minuten Rühren werden 9.98 mL (96.30 mmol) Cyclohexanon schnell zugegeben. Die Reaktionsmischung wird auf Raumtemperatur erwärmt, zwei Stunden gerührt und mit Ammoniumchlorid-Lösung versetzt. Die wässrige Phase wird abgetrennt und mit Ethylacetat erschöpfend extrahiert. Die vereinigten organischen Phasen werden mit Natriumchlorid-Lösung gewaschen, mit Natriumsulfat getrocknet und eingeengt. Säulenchromatographie (Kieselgel, Hexan/Ethylacetat = 20:1) liefert das Produkt als bräunliches Öl. Ausbeute: 5.20 g (29%); R_{f} = 0.26 (Kieselgel, Hexan/Ethylacetat =10:1); ESI-MS: [M+H-H2O]⁺ = 204.

### b) 1-(2-Amino-phenyl)-cyclohexanol

5.20 g (16.45 mmol) 1-(2-Nitro-phenyl)-cyclohexanol in 70 mL Ethanol werden in Gegenwart von Raney-Nickel bei Raumtemperatur und 3 bar Wasserstoffdruck 4 Stunden hydriert. Der Katalysator wird über Celite abfiltriert und das Filtrat im Vakuum eingeengt. Der Rückstand wird aus Hexan ausgefällt. Ausbeute: 1.53 g (49%); R_{f} = 0.38 (Kieselgel, Hexan/Ethylacetat = 4:1); ESI-MS: [M+H-H₂O]⁺ = 174.

### c) {3-[2-(1-Hydroxy-cyclohexyl)-phenylaminol]-1,1-dimethyl-propyl}-carbaminsäure-tert-butylester

Die Verbindung wird analog Zwischenprodukt 1b aus 1-(2-Amino-phenyl)-cyclohexanol und (1,1-Dimethyl-3-oxo-propyl)-carbaminsäure-tert-butylester erhalten. Säulenchromatographie (Kieselgel, Hexan/Ethylacetat = 7:1) liefert das Produkt in Form eines farblosen Öls. Ausbeute: 2.65 g (66%); R_{f} = 0.50 (Kieselgel, Hexan/Ethylacetat = 4:1).

### d){1,1-Dimethyl-3-[spiro(cyclohexan-1,4'-2H-3',1'-benzoxazin)-2'-oxo-1-yl]-propyl}-carbaminsäure-tert-butylester

Herstellung analog Zwischenprodukt 1c aus {3-[2-(1-Hydroxy-cyclohexyl)-phenylamino]-1,1-dimethyl-propyl -carbaminsäure-tert-butylester. Ausbeute: 2.60 g (92%); R_{f} = 0.38 (Kieselgel, Hexan/Ethylacetat 4:1).

### e) 1-(3-Amino-3-methyl-butyl)-spiro(cyclohexan-1,4'-2H-3',1'-benzoxazin)-2'-on

Herstellung analog Zwischenprodukt 1d aus [1,1-Dimethyl-3-(spiro(cyclohexan-1,4'-2H-3',1'-benzoxazin)-2'-oxo-1-yl)-propyl]-carbaminsäure-tert-butylester. Ausbeute: 1.80 g (92%); R_{f} = 0.10 (Kieselgel, Dichlormethan/Methanol/Ammoniak = 95:5:0.5); ESI-MS: [M+H]⁺ = 303.

### Zwischenprodukt 6: 1-(3-Amino-3-methyl-butyl)-4,4-diethyl-8-methoxy-1,4-dihydro-benzo[d][1,3]oxazin-2-on

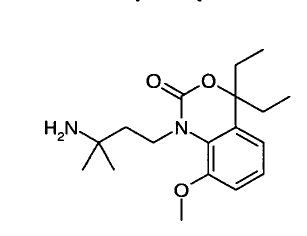

### a) 3-(2-Amino-3-methoxy-phenyl)-pentan-3-ol

Das Produkt wird analog Zwischenprodukt 1a durch Reaktion von 2-Amino-3-methoxybenzoesäuremethylester und Ethylmagnesiumbromid in Dichlormethan bei - 78 °C ? RT erhalten. Ausbeute: 5.20 g (92%); HPLC-MS: Rₜ = 12.85 Min. (Methode A); ESI-MS: [M+H]⁺ = 210.

### b) {3-[2-(1-Ethyl-1-hydroxy-propyl)-6-methoxy-phenylamino]-1,1-dimethyl-propyl}-carbaminsäure-tert-butylester

Das Produkt wird analog Zwischenprodukt 1b ausgehend von 3-(2-Amino-3-methoxyphenyl)-pentan-3-ol und (1,1-Dimethyl-3-oxo-propyl)-carbaminsäure-tert-butylester erhalten. Das Rohprodukt wird mittels Säulenchromatographie (Kieselgel, Cyclohexan/Ethylacetat = 4:1) gereinigt. Ausbeute: 4.60 g (47%).

### c) [3-(4,4-Diethyl-8-methoxy-2-oxo-4H-benzo[d][1,3]oxazin-1-yl)-1,1-dimethyl-propyl]-carbaminsäure-tert-butylester

Das Produkt wird analog Zwischenprodukt 1c ausgehend von {3-[2-(1-Ethyl-1-hydroxypropyl)-6-methoxy-phenylamino]-1,1-dimethyl-propyl-carbaminsäure-tert-butylester erhalten. Ausbeute: 4.60 g (94%).

### d) 1-(3-Amino-3-methyl-butyl)-4,4-diethyl-8-methoxy-1,4-dihydro-benzo[d][1,3]oxazin-2-on

Das Produkt wird analog Zwischenprodukt 1d ausgehend von [3-(4,4-Diethyl-8-methoxy-2-oxo-4*H*-benzo[*d*][1,3]oxazin-1-yl)-1,1-dimethyl-propyl]-carbaminsäure-tert-butylester als freie Base hergestellt. Ausbeute: 3.00 g (93%); ESI-MS: [M+H]⁺ = 321.

### Zwischenprodukt 7: 1-(3-Amino-3-methyl-butyl)-4,4-diethyl-6-fluor-1,4-dihydro-benzo[d][1,3]oxazin-2-on

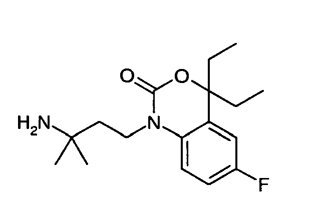

### a) 3-(2-Amino-5-fluor-phenyl)-pentan-3-ol

Herstellung analog Zwischenprodukt 1a aus 2-Amino-5-fluor-benzoesäuremethylester und Ethylmagnesiumbromid. Das erhaltene Produkt wird mittels Chromatographie (Kieselgel, Cyclohexan/Ethylacetat = 8:1) gereinigt. Ausbeute: 6.00 g (74%).

### b) {3-[2-(1-Ethyl-1-hydroxy-propyl)-4-fluor-phenylamino]-1,1-dimethyl-propyl}-carbaminsäure-tert-butylester

Das Produkt wird analog Zwischenprodukt 1b ausgehend von 3-(2-Amino-5-fluor-phenyl)-pentan-3-ol und (1,1-Dimethyl-3-oxo-propyl)-carbaminsäure-tert-butylester erhalten. Das Rohprodukt wird mittels Säulenchromatographie (Kieselgel, Hexan/Ethylacetat = 6:1 ? 2:1) gereinigt. Ausbeute: 4.50 g (41 %).

### c) [3-(4,4-Diethyl-6-fluor-2-oxo-4H-benzo[d][1,3]oxazin-1-yl)-1,1-dimethyl-propyl]-carbaminsäure-tert-butylester

Herstellung in Analogie zu Zwischenprodukt 1c aus {3-[2-(1-Ethyl-1-hydroxy-propyl)-4-fluor-phenylamino]-1,1-dimethyl-propyl}-carbaminsäure-tert-butylester. Abweichend wird auf eine säulenchromatographische Reinigung verzichtet. Farbloses Öl. Ausbeute: 4.8 g.

### d) 1-(3-Amino-3-methyl-butyl)-4,4-diethyl-6-fluor-1,4-dihydro-benzo[d][1,3]oxazin-2-on

Die Zielverbindung wird als freie Base analog Zwischenprodukt 1d aus [3-(4,4-Diethyl-6-fluor-2-oxo-4H-benzo[d] [1,3]oxazin-1-yl)-1,1-dimethyl-propyl]-carbaminsäure-tert-butylester hergestellt. Ausbeute: 3.00 g (99%).

### Zwischenprodukt 7: 1-(3-Amino-3-methyl-butyl)-4,4-diethyl-6-methoxy-1,4-dihydro-benzo[d][1,3]oxazin-2-on

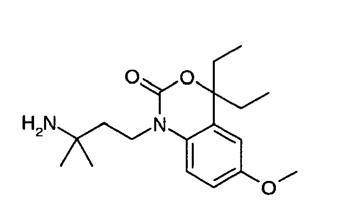

### a) 3-(2-Amino-5-methoxy-phenyl)-pentan-3-ol

Das Produkt wird aus der Umsetzung von 4.00 g (22 mmol) 2-Amino-5-methoxybenzoesäuremethylester mit 5 Äquivalenten Ethylmagnesiumbromid in Dichlormethan bei - 78 °C -> RT erhalten. Braunes Öl. Ausbeute: 4.47 g (97%).

### b) {3-[2-(1-Ethyl-1-hydroxy-propyl)-4-methoxy-phenylaminol-1,1-dimethyl-propyl}-carbaminsäure-tert-butylester

Herstellung analog Zwischenprodukt 1b aus 4.45 g (21 mmol) 3-(2-Amino-5-methoxyphenyl)-pentan-3-ol und 5.66 g (28 mmol) (1,1-Dimethyl-3-oxo-propyl)-carbaminsäure-tert-butylester. Braunes Öl. Ausbeute: 6.00 g (72%).

### c) [3-(4,4-Diethyl-6-methoxy-2-oxo-4H-benzo[d]|1,3]oxazin-1-yl)-1,1-dimethyl-propyl]-carbaminsäure-tert-butylester

Das Produkt wird analog Zwischenprodukt 1c aus 6.00 g (15.2 mmol) {3-[2-(1-Ethyl-1-hydroxy-propyl)-4-methoxy-phenylamino]-1,1-dimethyl-propyl -carbaminsäure-tert-butylester hergestellt. Gelbes Öl. Ausbeute: 3.10 g (48%).

### d) 1-(3-Amino-3-methyl-butyl)-4,4-diethyl-6-methoxy-1,4-dihydro-benzo[d][1,3]oxazin-2-on

Hergestellt analog Zwischenprodukt 1d aus 3.10 g (8.5 mmol) [3-(4,4-Diethyl-6-methoxy-2-oxo-4*H*-benzo[*d*] [1,3]oxazin-1-yl)-1,1-dimethyl-propyl]-carbaminsäure-tert-butylester. Das Produkt wird als freie Base isoliert und nicht in ein Hydrochloridsalz überführt. Gelbes Öl. Ausbeute: 2.20 g (98%).

### Beispiel 7: N-(5-(2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid

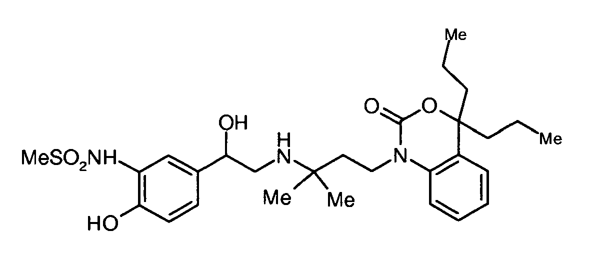

### a) N-(2-Benzyloxy-5-{2-[1,1-dimethyl-3-(2-oxo-4,4-dipropyl-4H-benzo[d][1,3]oxazin-1-yl)-propylamino]-1-hydroxy-ethyl}phenyl)-methansulfonamid

Zu einer Lösung von 200 mg (0.564 mmol) 1-(3-Amino-3-methyl-butyl)-4,4-dipropyl-1,4-dihydro-benzo[*d*][1,3]oxazin-2-on hydrochlorid in 5 mL THF werden bei Raumtemperatur unter Stickstoffatmosphäre 86 µl (0.619 mmol) Triethylamin gegeben. Man läßt 30 Minuten rühren, setzt 218 mg (0.575 mmol) N-[2-Benzyloxy-5-(2-ethoxy-2-hydroxy-acetyl)-phenyl]-methansulfonamid zu und rührt weitere 2 Stunden bei Raumtemperatur. Das Gemisch wird auf 10°C gekühlt, mit 51 mg (2.34 mmol) Lithiumborhydrid versetzt, auf Raumtemperatur erwärmt und eine Stunde gerührt. Es wird erneut auf 10°C gekühlt und mit 15 mL Wasser und 20 mL Dichlormethan verdünnt. Die wässrige Phase wird abgetrennt und mit Dichlormethan extrahiert. Die vereinigten organischen Phasen werden mit Natriumsulfat getrocknet und im Vakuum eingeengt. Der Rückstand wird in 8 mL Ethylacetat gelöst und durch Zugabe gesättigter Salzsäure in Ethylacetat auf pH 2 angesäuert. Der sich bildende Niederschlag wird abfiltriert, mit Ethylacetat gewaschen und eingeengt. Ausbeute: 260 mg (67%, Hydrochlorid), HPLC: Rₜ = 19.8 Minuten (Methode A).

### b) N-(5-{2-[1,1-Dimethyl-3-(2-oxo-4,4-dipropyl-4H-benzo[d] [1,3]oxazin-1-yl)-propylamino]-1-hydroxy-ethyl}-2-hydroxy-phenyl)-methansulfonamid

260 mg (0.386 mmol) N-(2-Benzyloxy-5-{2-[1,1-dimethyl-3-(2-oxo-4,4-dipropyl-4H-benzo[d][1,3]oxazin-1-yl)-propylamino]-1-hydroxy-ethyl}-phenyl)-methansulfonamid hydrochlorid in 8 mL Methanol werden in Gegenwart von 26 mg Palladium auf Kohle (10%ig) bei Raumtemperatur hydriert. Der Katalysator wird über Celite abfiltriert und mit Methanol gewaschen. Das Filtrat wird im Vakuum eingeengt und der Rückstand in Diethylether verrührt. Ausbeute: 120 mg (53%, Hydrochlorid); Massenspektroskopie: [M+H]⁺ = 548; HPLC: Rₜ = 14.7 Minuten (Methode A).

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 8: N-[5-(2-{1,1-Dimethyl-3-[spiro(cyclohexan-1,4'-2H-3',1'-benzoxazin)-2'-oxo-1-yl]-propylamino}-1-hydroxy-ethyl)-2-hydroxy-phenyl]-methansulfonamid

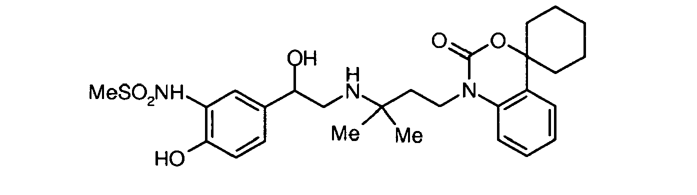

### a) N-[2-Benzyloxy-5-(2-{3-[spiro(cyclohexan-1,4'-2H-3',1'-benzoxazin)-2'-oxo-1-yl]-1,1-dimethyl-propylamino}-1-hydroxy-ethyl]-phenyl]-methansulfonamid

Herstellung in Analogie zu dem für Beispiel 7a beschriebenen Verfahren aus 250 mg (0.66 mmol) N-[2-Benzyloxy-5-(2-ethoxy-2-hydroxy-acetyl)-phenyl]-methansulfonamid und 200 mg (0.66 mmol) 1-(3-Amino-3-methyl-butyl)-spiro(cyclohexan-1,4'-2H-3',1'-benzoxazin)-2'-on. Abweichend wird das als Hydrochlorid anfallende Produkt noch chromatographisch gereinigt (Kieselgel, Dichlormethan/Methanol = 50:1). Ausbeute: 190 mg (46%), HPLC: Rₜ = 17.8 Minuten (Methode A).

### b) N-[5-(2-{1,1-Dimethyl-3-[spiro(cyclohexan-1,4'-2H-3',1'-benzoxazin)-2'-oxo-1-yl]-propylamino}-1-hydroxy-ethyl)-2-hydroxy-phenyl]-methansulfonamid

190 mg (0.31 mmol) N-[2-Benzyloxy-5-(2-{3-[spiro(cyclohexan-1,4'-2H-3',1'-benzoxazin)-2'-oxo-1-yl]-1,1-dimethyl-propylamino}-1-hydroxy-ethyl]-phenyl]-methansulfonamid werden analog Beispiel 7b hydriert. Nach dem Abtrennen des Katalysators wird das Filtrat vom Lösungsmittel befreit, mit 8 mL Ethylacetat versetzt und durch Zugabe von Salzsäure in Ethylacetat auf pH 2 angesäuert. Das Lösungmittel wird abdestilliert und der Rückstand in Diethylether verrührt und filtriert. Ausbeute: 40 mg (23%, Hydrochlorid); Massenspektroskopie: [M+H]⁺ = 532; HPLC: Rₜ = 11.8 Minuten (Methode A).

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 9: N-[5-(2-{1,1-Dimethyl-3-[spiro(cyclopropyl-1,4'-2H-3',1'-benzoxazin)-2'-oxo-1-yl]-propylamino}-1-hydroxy-ethyl)-2-hydroxy-phenyl]-methansulfonamid

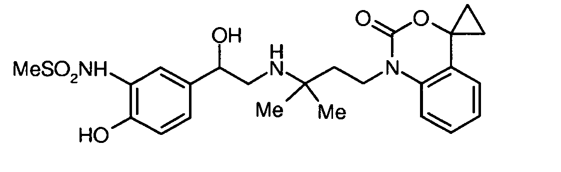

### a) N-[2-Benzyloxy-5-(2-{3-[spiro(cyclopropyl-1,4'-2H-3',1'-benzoxazin)-2'-oxo-1-yl]-1,1-dimethyl-propylamino}-1-hydroxy-ethyl]-phenyl]-methansulfonamid

292 mg (0.77 mmol) N-[2-Benzyloxy-5-(2-ethoxy-2-hydroxy-acetyl)-phenyl]-methansulfonamid und 200 mg (0.77 mmol) 1-(3-Amino-3-methyl-butyl)-spiro(cyclopropyl-1,4'-2H-3',1'-benzoxazin)-2'-on werden analog Beispiel 7a umgesetzt und aufgearbeitet. Das Rohprodukt wird mit 8 mL Ethylacetat versetzt und mit Salzsäure in Ethylacetat auf pH 2 angesäuert. Das Lösungsmittel wird abdestilliert und der Rückstand in Diethylether verrührt. Weißer Feststoff. Ausbeute: 400 mg (84%, Hydrochlorid), HPLC: Rₜ = 15.2 Minuten (Methode A).

### b) N-[5-(2-{1,1-Dimethyl-3-[spiro(cyclopropyl-1,4'-2H-3',1'-benzoxazin)-2'-oxo-1-yl]-propylamino}-1-hydroxy-ethyl)-2-hydroxy-phenyl]-methansulfonamid

Das Produkt wird analog Beispiel 1b aus 400 mg (0.65 mmol) N-[2-Benzyloxy-5-(2-{3-[spiro(cyclopropyl-1,4'-2H-3',1'-benzoxazin)-2'-oxo-1-yl]-1,1-dimethyl-propylamino }-1-hydroxy-ethyl]-phenyl]-methansulfonamid hydrochlorid hergestellt. Ausbeute: 230 mg (67%, Hydrochlorid); Massenspektroskopie: [M+H]⁺ = 490; HPLC: Rₜ = 8.9 Minuten (Methode A).

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 10: N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid

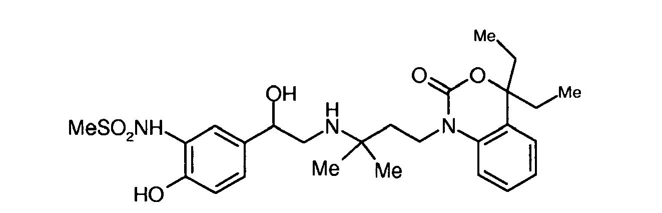

379 mg (1 mmol) N-[2-Benzyloxy-5-(2-ethoxy-2-hydroxy-acetyl)-phenyl]-methansulfonamid und 290 mg (1 mmol) 1-(3-Amino-3-methyl-butyl)-4,4-diethyl-1,4-dihydro-benzo[d][1,3]oxazin-2-on werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Dichlormethan/Methanol (15:1) nachgewaschen und eingeengt. Das so erhaltene Rohprodukt wird chromatographisch (Dichlormethan mit 0-10% Methanol/Ammoniak = 9:1) gereinigt. Der so erhaltene Benzylether wird in 10 mL Methanol gelöst und mit Palladium auf Kohle als Katalysator bei 1 bar Wasserstoffdruck hydriert. Anschließend wird der Katalysator abfiltriert und das Filtrat eingeengt. Weißer Feststoff. Ausbeute: 338 mg (65% über 2 Stufen); Massenspektroskopie: [M+H]⁺ = 520.

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu. Der Drehwert von (R)-N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid hydrochlorid (kokristallisiert mit einem Molekül Aceton) beträgt -28.8° (c = 1%, in Methanol bei 20°C).

### Beispiel 11: N-(5-{2-[3-(4,4-Diethyl-6-fluoro-2-oxo-4H-benzo[d][1,3]oxazin-1-yl)-1,1-dimethyl-propylaminol-1-hydroxy-ethyl}-2-hydroxy-phenyl)-methansulfonamid

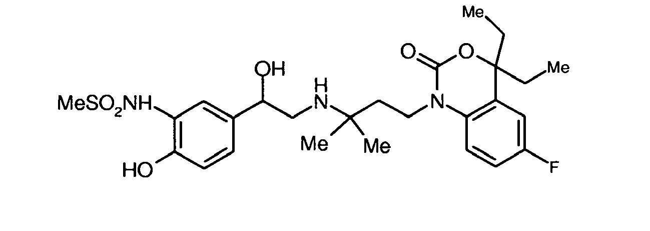

### a) N-(2-Benzyloxy-5-{2-[3-(4,4-diethyl-6-fluor-2-oxo-4H-benzo[d][1,3]oxazin-1-yl)-1,1-dimethyl-propylamino]-1-hydroxy-ethyl}-phenyl)-methansulfonamid

Umsetzung von 246 mg (0.65 mmol) N-[2-Benzyloxy-5-(2-ethoxy-2-hydroxy-acetyl)-phenyl]-methansulfonamid und 200 mg (0.65 mmol) 1-(3-Amino-3-methyl-butyl)-4,4-diethyl-6-fluor-1,4-dihydro-benzo[D][1,3]oxazin-2-on analog Beispiel 7a. Abweichend wird auf die Herstellung des Hydrochlorids verzichtet. Stattdessen wird die freie Base chromatographisch gereinigt (Reverse Phase, Acetonitril/Wasser-Gradient mit 0.1% Trifluoressigsäure). Ausbeute: 180 mg (Trifluoracetat), HPLC: Rₜ = 17.4 Minuten (Methode A).

### b) N-(5-{2-[3-(4,4-Diethyl-6-fluor-2-oxo-4H-benzo[d][1,3]oxazin-1-yl)-1,1-dimethyl-propylamino]-1-hydroxy-ethyl}-2-hydroxy-phenyl)-methansulfonamid

175 mg - N-(2-Benzyloxy-5-{2-[3-(4,4-diethyl-6-fluor-2-oxo-4H-benzo[d][1,3]oxazin-1-yl)-1,1-dimethyl-propylamino]-1-hydroxy-ethyl}-phenyl)-methansulfonamid trifluoracetat in 9 mL Methanol werden in Gegenwart von 40 mg Raney-Nickel bei Raumtemperatur und 3 bar Wasserstoffdruck hydriert. Der Katalysator wird abfiltriert und das Filtrat vom Lösungsmittel befreit. Weißer Feststoff. Ausbeute: 131 mg (Trifluoracetat); Massenspektroskopie: [M+H]⁺ = 538.

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 12: N-(5-{2-[3-(4,4-Diethyl-7-fluoro-2-oxo-4H-benzo[d][1,3]oxazin-1-yl)-1 dimethyl-propylamino]-1-hydroxy-ethyl}-2-hydroxy-phenyl)-methansulfonamid

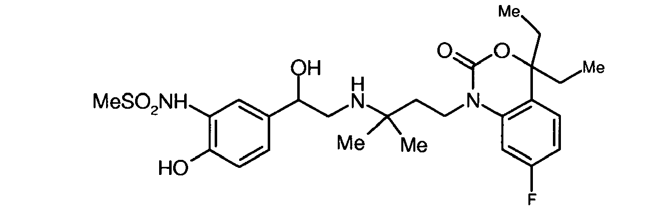

### a) N-(2-Benzyloxy-5-{2-[3-(4,4-diethyl-7-fluor-2-oxo-4H-benzo[d][1,3]oxazin-1-yl)-1,1-dimethyl-propylamino]-1-hydroxy-ethyl}-phenyl)-methansulfonamid

246 mg (0.65 mmol) N-[2-Benzyloxy-5-(2-ethoxy-2-hydroxy-acetyl)-phenyl]-methansulfonamid und 200 mg (0.65 mmol) 1-(3-Amino-3-methyl-butyl)-4,4-diethyl-7-fluor-1,4-dihydro-benzo[D][1,3]oxazin-2-on werden analog Beispiel 7a umgesetzt und aufgearbeitet. Abweichend wird auf die Herstellung des Hydrochlorids verzichtet und die freie Base wird chromatographisch gereinigt (Reverse Phase, Acetonitril/Wasser-Gradient mit 0.1 % Trifluoressigsäure). Ausbeute: 220 mg (Trifluoracetat), HPLC: Rₜ = 17.7 Minuten (Methode A).

### b) N-(5-{2-[3-(4,4-Diethyl-7-fluor-2-oxo-4H-benzo[d][1,3]oxazin-1-yl)-1,1-dimethyl-propylamino]-1-hydroxy-ethyl}-2-hydroxy-phenyl)-methansulfonamid

Herstellung analog Beispiel 11b aus 210 mg N-(2-Benzyloxy-5-{2-[3-(4,4-diethyl-7-fluor-2-oxo-4H-benzo[d][1,3]oxazin-1-yl)-1,1-dimethyl-propylamino]-1-hydroxy-ethyl} - phenyl)-methansulfonamid trifluoracetat. Grauer Feststoff. Ausbeute: 154 mg (Trifluoracetat); Massenspektroskopie: [M+H]⁺ = 538.

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 13: N-(5-{2-[3-(4,4-Diethyl-8-methoxy-2-oxo-4H-benzo[d][1,3]oxazin-1-yl)-1,1 - dimethyl-propylamino]-1-hydroxy-ethyl}1-2-hydroxy-phenyl)-methansulfonamid

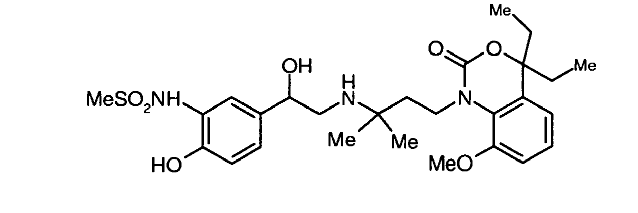

### a) N-(2-Benzyloxy-5-{2-[3-(4,4-diethyl-8-methoxy-2-oxo-4H-benzo[d][1,3]oxazin-1-yl)-1,1-dimethyl-propylamino]-1-hydroxy-ethyl}-phenyl)-methansulfonamid

Umsetzung von 237 mg (0.625 mmol) N-[2-Benzyloxy-5-(2-ethoxy-2-hydroxy-acetyl)-phenyl]-methansulfonamid und 200 mg (0.624 mmol) 1-(3-Amino-3-methyl-butyl)-4,4-diethyl-8-methoxy-1,4-dihydro-benzo[d][1,3]oxazin-2-on analog Beispiel 7a. Das Rohprodukt wird in Ethylacetat gelöst und mit Salzsäure in Ethylacetat auf pH 2 angesäuert. Das Lösungsmittel wird abdestilliert und der Rückstand in Diethylether verrührt. Anschließend wird das so erhaltene Hydrochlorid (330 mg) weiter chromatographisch gereinigt. Ausbeute: 90 mg (Trifluoracetat), HPLC: Rₜ = 17.6 Minuten (Methode A).

### b) N-(5-{2-[3-(4,4-Diethyl-8-methoxy-2-oxo-4H-benzo[d][1,3]oxazin-1-yl)-1,1-dimethyl-propylamino]-1-hydroxy-ethyl}-2-hydroxy-phenyl)-methansulfonamid

80 mg (0.118 mmol) N-(2-Benzyloxy-5-{2-[3-(4,4-diethyl-8-methoxy-2-oxo-4H-benzo[d][1,3]oxazin-1-yl)-1,1-dimethyl-propylamino]-1-hydroxy-ethyl}-phenyl)-methansulfonamid trifluoracetat werden analog Beispiel 11b hydriert. Beiger Feststoff. Ausbeute: 70 mg (Trifluoracetat); Massenspektroskopie: [M+H]⁺ = 550.

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

### Beispiel 14: N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid

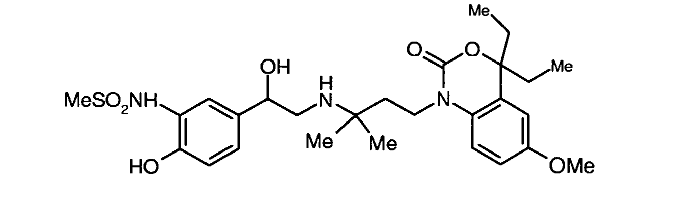

### a) N-(2-Benzyloxy-5-{2-[3-(4,4-diethyl-6-methoxy-2-oxo-4H-benzo[d][1,3]oxazin-1-yl)-1,1-dimethyl-propylamino]-1-hydroxy-ethyl}-phenyl)-methansulfonamid

235 mg (0.619 mmol) N-[2-Benzyloxy-5-(2-ethoxy-2-hydroxy-acetyl)-phenyl]-methansulfonamid und 200 mg (0.624 mmol) 1-(3-Amino-3-methyl-butyl)-4,4-diethyl-6-methoxy-1,4-dihydro-benzo[d][1,3]oxazin-2-on werden analog Beispiel 7a umgesetzt. Abweichend wird das Rohprodukt nicht als Hydrochlorid ausgefällt, sondern chromatographisch gereinigt (Reverse Phase, Acetonitril/Wasser-Gradient mit 0.1% Trifluoressigsäure). Ausbeute: 150 mg (Trifluoracetat), HPLC: Rₜ = 16.9 Minuten (Methode A).

### b) N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid

Die Zielverbindung wird aus N-(2-Benzyloxy-5-{2-[3-(4,4-diethyl-6-methoxy-2-oxo-4H-benzo[d][1,3]oxazin-1-yl)-1,1-dimethyl-propylamino]-1-hydroxy-ethyl}-phenyl)-methansulfonamid trifluoracetat analog Beispiel 11b hergestellt. Grauer Feststoff (Trifluoracetat). Massenspektroskopie: [M+H]⁺ = 550.

Die (R)- und (S)-Enantiomere dieses Ausführungsbeispiels können nach gängigen, im Stand der Technik bekannten Methoden erhalten werden. Dem (R)-Enantiomer dieses Ausführungsbeispiels kommt erfindungsgemäß besondere Bedeutung zu.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäßen Applikation der Verbindungen der Formel 1 zur Therapie der vorstehend genannten Atemwegserkrankungen werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erfindungsgemäß einsetzbare Inhalationspulver können **1** entweder allein oder im Gemisch mit geeigneten physiologisch unbedenkliche Hilfsstoffen enthalten.
Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen erfolgen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure und Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.
Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1**, als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:

### Pharmazeutische Formulierungsbeispiele

| A) | Tabletten | pro Tablette |
|---|---|---|
| | Wirkstoff **1** | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff **1** | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | **W**irkstoff **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff **1** | 0,005 |
| | Sorbitantrioleat | 0,1 |
| | Monofluortrichlormethan und TG134a : TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff **1** | 333.3 mg |
| | Benzalkoniumchlorid | 10.0 mg |
| | EDTA | 50.0 mg |
| | HCl (1n) | ad pH 3.4 |

Diese Lösung kann in üblicher Art und Weise hergestellt werden.

| F) | Inhalationspulver | |
|---|---|---|
| | Wirkstoff **1** | 12 µg |
| | Lactose Monohydrat | ad 25 mg |

Die Herstellung des Inhalationspulvers erfolgt in üblicher Art und Weise durch Mischen der einzelnen Bestandteile.

## Patentansprüche

1. Verwendung von Verbindungen der allgemeinen Formel **1** 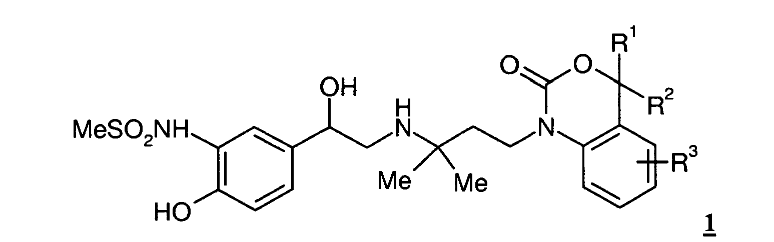 worin
R¹ und R² gleich oder verschieden, Wasserstoff, Halogen, -C₁-C₄-Alkyl oder gemeinsam -C₁-C₆-Alkylen und
R³ Wasserstoff, Halogen, OH, C₁-C₄-Alkyl oder -O-C₁-C₄-Alkyl;
bedeuten, zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

2. Verwendung von Verbindungen der allgemeinen Formel **1** nach Anspruch 1, worin
R¹ und R² gleich oder verschieden, Wasserstoff, Fluor, Chlor, Methyl, Ethyl, Propyl, Butyl oder gemeinsam -CH₂-CH₂-, -CH₂-CH₂-CH₂-, -CH₂-CH₂-CH₂-CH₂- oder -CH₂-CH₂-CH₂-CH₂-CH₂-;
R³ Wasserstoff, Fluor, Chlor, OH, Methyl, Ethyl, Methoxy, oder Ethoxy bedeuten.

3. Verwendung von Verbindungen der allgemeinen Formel **1** nach Anspruch 1, worin
R¹ und R² gleich oder verschieden, Wasserstoff, Methyl, Ethyl, Propyl oder gemeinsam -CH₂-CH₂-, -CH₂-CH₂-CH₂-, -CH₂-CH₂-CH₂-CH₂- oder -CH₂-CH₂-CH₂-CH₂-CH₂-;
R³ Wasserstoff, Fluor, OH, Methyl oder Methoxy bedeuten.

4. Verwendung von Verbindungen der allgemeinen Formel **1** nach einem der Ansprüche 1 bis 3 zur Herstellung eines Arzneimittels zur Behandlung von Obstruktiven Lungenerkrankungen die ausgewählt sind aus der Gruppe bestehend aus Asthma Bronchiale, pädiatrisches Asthma, schweres Asthma, akuter Asthma-Anfall, chronische Bronchitis und COPD, wobei die Verwendung zur Herstellung eines Arzneimittels zur Behandlung von Asthma Bronchiale oder zur Behandlung von COPD erfindungsgemäß besonders bevorzugt ist.

5. Verwendung von Verbindungen der allgemeinen Formel **1** nach einem der Ansprüche 1 bis 3 zur Herstellung eines Arzneimittels zur Behandlung von Lungenemphysemen, die ihren Ursprung haben in COPD oder α1-Proteinase-Inhibitor-Mangel.

6. Verwendung von Verbindungen der allgemeinen Formel **1** nach einem der Ansprüche 1 bis 3 zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

7. Verwendung von Verbindungen der allgemeinen Formel **1** nach einem der Ansprüche 1 bis 3 zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

8. Verwendung von Verbindungen der allgemeinen Formel **1** nach einem der Ansprüche 1 bis 3 zur Herstellung eines Arzneimittels zur Behandlung von Zystischer Fibrose bzw. Mukoviszidose, Bronchiektasen oder ARDS (adult respiratory distress syndrom).

9. Verwendung von Verbindungen der allgemeinen Formel **1** nach einem der Ansprüche 1 bis 3 zur Herstellung eines Arzneimittels zur Behandlung von Bronchitiden, wie beispielsweise Bronchitis aufgrund bakterieller oder viraler Infektion, Allergische Bronchitis und Toxische Bronchitis.

10. Verwendung von Verbindungen der allgemeinen Formel **1** nach einem der Ansprüche 1 bis 3 zur Herstellung eines Arzneimittels zur Behandlung von Lungenödemen, beispielsweise toxischer Lungenödeme nach Aspiration oder Inhalation von toxischen Substanzen und Fremdstoffen.

11. Verwendung von Verbindungen der allgemeinen Formel **1** nach einem der Ansprüche 1 bis 10, worin die Verbindungen der Formel **1** in Form der einzelnen optischen Isomeren, Mischungen der einzelnen Enantiomeren gegebenenfalls Diastereomeren oder Racemate, bevorzugt in Form der enantiomerenreinen gegebenenfalls diastereomerenreinen Verbindungen vorliegen.

12. Verwendung von Verbindungen der allgemeinen Formel **1** nach einem der Ansprüche 1 bis 10, worin die Verbindungen der Formel **1** in Form der freien Basen oder in Form der Säureadditionssalze mit pharmakologisch unbedenklichen Säuren sowie gegebenenfalls in Form der Solvate und/oder Hydrate vorliegen.

13. Verbindungen der Formel **1**, 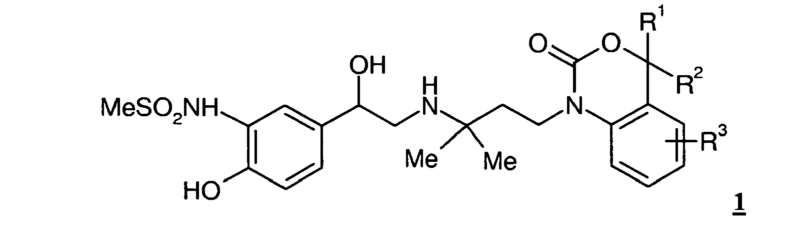 worin
R¹ und R² gleich oder verschieden, bevorzugt gleich Ethyl oder Propyl, oder gemeinsam -CH₂-CH₂-, -CH₂-CH₂-CH₂-, -CH₂-CH₂-CH₂-CH₂- oder -CH₂-CH₂-CH₂-CH₂-CH₂- und
R³ Wasserstoff, Fluor, Chlor, OH, Methyl, Ethyl, Methoxy oder Ethoxy bedeuten.

14. Verbindungen der Formel **1** nach Anspruch 13, in denen
R¹ und R² gleich oder verschieden, bevorzugt gleich, Ethyl oder Propyl, oder gemeinsam -CH₂-CH₂-, -CH₂-CH₂-CH₂-CH₂- oder -CH₂-CH₂-CH₂-CH₂-CH₂- bedeuten und in denen
R³ Wasserstoff, Fluor, OH, Methyl oder Methoxy, bevorzugt Wasserstoff sein kann.

15. Verbindungen der Formel **1** nach Anspruch 13 oder 14 die ausgewählt sind aus der Gruppe bestehend aus
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid;
- N-[5-(2-{1,1-Dimethyl-3-[spiro(cyclohexan-1,4'-2H-3',1'-benzoxazin)-2'-oxo-1-yl]-propylamino}-1-hydroxy-ethyl)-2-hydroxy-phenyl]-methansulfonamid;
- N-[5-(2-{1,1-Dimethyl-3-[spiro(cyclopropyl-1,4'-2H-3',1'-benzoxazin)-2'-oxo-1-yl]-propylamino}-1-hydroxy-ethyl)-2-hydroxy-phenyl]-methansulfonamid;
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-7-fluoro-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-8-methoxy-2-oxo-4H-benzo[d][1,3]oxazin-1-yl)-1,1-dimethyl-propylamino]-1-hydroxy-ethyl}-2-hydroxy-phenyl)-methansulfonamid;
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid

16. Verbindungen der allgemeinen Formel **1** nach einem der Ansprüche 13 bis 15, worin die Verbindungen der Formel **1** in Form der einzelnen optischen Isomeren, Mischungen der einzelnen Enantiomeren gegebenenfalls Diastereomeren oder Racemate, bevorzugt in Form der enantiomerenreinen gegebenenfalls diastereomerenreinen Verbindungen vorliegen.

17. Verbindungen der allgemeinen Formel **1** nach einem der Ansprüche 13 bis 15, worin die Verbindungen der Formel **1** in Form der freien Basen oder in Form der Säureadditionssalze mit pharmakologisch unbedenklichen Säuren sowie gegebenenfalls in Form der Solvate und/oder Hydrate vorliegen.

18. Verbindungen der allgemeinen Formel **1** gemäß einem der Ansprüche 13 bis 17 als Arzneimittel.

19. Pharmazeutischen Formulierung, **gekennzeichnet durch** einen Gehalt einer Verbindung der Formel **1** gemäß einem der Ansprüche 13 bis 17.

## Claims

1. Use of compounds of general formula **1** 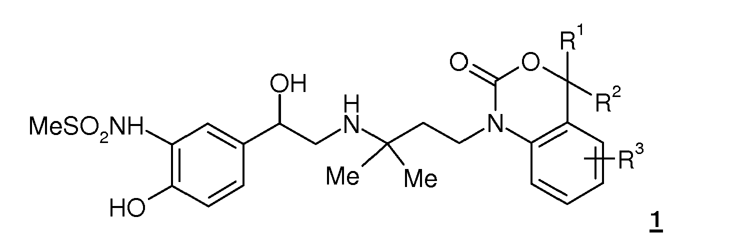 wherein
R¹ and R², which may be identical or different, denote hydrogen, halogen, -C₁-C₄-alkyl or together denote -C₁-C₆-alkylene and
R³ denotes hydrogen, halogen, OH, C₁-C₄-alkyl or -O-C₁-C₄-alkyl;
for preparing a medicament for the treatment of respiratory complaints which are selected from the group comprising obstructive pulmonary diseases of various origins, pulmonary emphysema of various origins, restrictive pulmonary diseases, interstitial pulmonary diseases, cystic fibrosis, bronchitis of various origins, bronchiectasis, ARDS (adult respiratory distress syndrome) and all forms of pulmonary oedema.

2. Use of compounds of general formula **1** according to claim 1, wherein
R¹ and R², which may be identical or different, denote hydrogen, fluorine, chlorine, methyl, ethyl, propyl, butyl or together denote -CH₂-CH₂-, -CH₂-CH₂-CH₂-, -CH₂-CH₂-CH₂-CH₂- or -CH₂-CH₂-CH₂-CH₂-CH₂-;
R³ denotes hydrogen, fluorine, chlorine, OH, methyl, ethyl, methoxy, or ethoxy.

3. Use of compounds of general formula **1** according to claim 1, wherein
R¹ and R², which may be identical or different, denote hydrogen, methyl, ethyl, propyl or together denote -CH₂-CH₂-, -CH₂-CH₂-CH₂-, -CH₂-CH₂-CH₂-CH₂- or -CH₂-CH₂-CH₂-CH₂-CH₂-;
R³ denotes hydrogen, fluorine, OH, methyl or methoxy.

4. Use of compounds of general formula **1** according to one of claims 1 to 3 for preparing a medicament for the treatment of obstructive pulmonary diseases selected from the group consisting of bronchial asthma, paediatric asthma, severe asthma, acute asthma attacks, chronic bronchitis and COPD, their use for preparing a medicament for the treatment of bronchial asthma or COPD being particularly preferred according to the invention.

5. Use of compounds of general formula **1** according to one of claims 1 to 3 for preparing a medicament for the treatment of pulmonary emphysema which has its origins in COPD or α1-proteinase inhibitor deficiency.

6. Use of compounds of general formula **1** according to one of claims 1 to 3 for preparing a medicament for the treatment of restrictive pulmonary diseases selected from the group consisting of allergic alveolitis, restrictive pulmonary diseases triggered by work-related noxious substances, such as asbestosis or silicosis, and restriction caused by lung tumours, such as for example lymphangiosis carcinomatosa, bronchoalveolar carcinoma and lymphomas.

7. Use of compounds of general formula **1** according to one of claims 1 to 3 for preparing a medicament for the treatment of interstitial pulmonary diseases selected from the group consisting of pneumonia caused by infections, such as for example infection by viruses, bacteria, fungi, protozoa, helminths or other pathogens, pneumonitis caused by various factors, such as for example aspiration and left heart insufficiency, radiation-induced pneumonitis or fibrosis, collagenoses, such as for example lupus erythematodes, systemic scleroderma or sarcoidosis, granulomatoses, such as for example Boeck's disease, idiopathic interstitial pneumonia or idiopathic pulmonary fibrosis (IPF).

8. Use of compounds of general formula **1** according to one of claims 1 to 3 for preparing a medicament for the treatment of cystic fibrosis or mucoviscidosis, bronchiectasis or ARDS (adult respiratory distress syndrome).

9. Use of compounds of general formula **1** according to one of claims 1 to 3 for preparing a medicament for the treatment of bronchitis, such as for example bronchitis caused by bacterial or viral infection, allergic bronchitis and toxic bronchitis.

10. Use of compounds of general formula **1** according to one of claims 1 to 3 for preparing a medicament for the treatment of pulmonary oedemas, for example toxic pulmonary oedemas after aspiration or inhalation of toxic substances and foreign substances.

11. Use of compounds of general formula **1** according to one of claims 1 to 10, wherein the compounds of formula **1** are present in the form of the individual optical isomers, mixtures of the individual enantiomers or optionally diastereomers or racemates, preferably in the form of the enantiomerically pure, optionally diastereomerically pure, compounds.

12. Use of compounds of general formula **1** according to one of claims 1 to 10, wherein the compounds of formula **1** are in the form of the free bases or in the form of the acid addition salts with pharmacologically acceptable acids as well as optionally in the form of the solvates and/or hydrates.

13. Compounds of formula **1**, 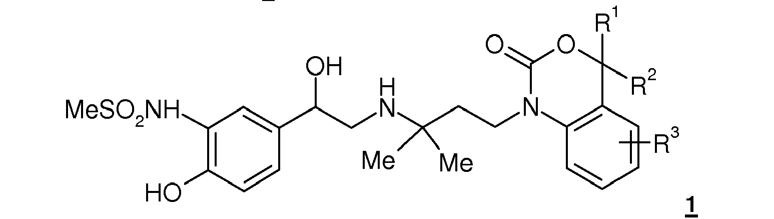 wherein
R¹ and R² which may be identical or different, preferably identical, denote ethyl or propyl, or together denote -CH₂-CH₂-, -CH₂-CH₂-CH₂-, -CH₂-CH₂-CH₂-CH₂- or -CH₂-CH₂-CH₂-CH₂-CH₂- and
R³ denotes hydrogen, fluorine, chlorine, OH, methyl, ethyl, methoxy or ethoxy.

14. Compounds of formula **1** according to claim 13, wherein
R¹ and R², which may be identical or different, preferably identical, denote ethyl or propyl, or together denote -CH₂-CH₂-, -CH₂-CH₂-CH₂-CH₂- or -CH₂-CH₂-CH₂-CH₂-CH₂- and wherein
R³ may denote hydrogen, fluorine, OH, methyl or methoxy, preferably hydrogen.

15. Compounds of formula **1** according to claim 13 or 14 which are selected from the group consisting of
- N-(5-{2-[1,1-dimethyl-3-(2-oxo-4,4-dipropyl-4H-benzo[d][1,3]oxazin-1-yl)-propylamino]-1-hydroxy-ethyl}-2-hydroxy-phenyl)-methanesulphonamide;
- N-[5-(2-{1,1-dimethyl-3-[spiro(cyclohexane-1,4'-2H-3',1'-benzoxazin)-2'-oxo-1-yl]-propylamino}-1-hydroxy-ethyl)-2-hydroxy-phenyl]-methanesulphonamide;
- N-[5-(2-{1,1-dimethyl-3-[spiro(cyclopropyl-1,4'-2H-3',1'-benzoxazin)-2'-oxo-1-yl]-propylamino}-1-hydroxy-ethyl)-2-hydroxy-phenyl]-methanesulphonamide;
- N-(5-{2-[3-(4,4-diethyl-2-oxo-4H-benzo[d][1,3]oxazin-1-yl)-1,1-dimethyl-propylamino]-1-hydroxy-ethyl]-2-hydroxy-phenyl)-methanesulphonamide;
- N-(5-{2-[3-(4,4-diethyl-6-fluoro-2-oxo-4H-benzo[d][1,3]oxazin-1-yl)-1,1-dimethyl-propylamino]-1-hydroxy-ethyl]-2-hydroxy-phenyl)-methanesulphonamide;
- N-(5-{2-[3-(4,4-diethyl-7-fluoro-2-oxo-4H-benzo[d][1,3]oxazin-1-yl)-1,1-dimethyl-propylamino]-1-hydroxy-ethyl}-2-hydroxy-phenyl)-methanesulphonamide;
- N-(5-{2-[3-(4,4-diethyl-8-methoxy-2-oxo-4H-benzo[d][1,3]oxazin-1-yl)-1,1-dimethyl-propylamino]-1-hydroxy-ethyl}-2-hydroxy-phenyl)-methanesulphonamide;
- N-(5-{2-[3-(4,4-diethyl-6-methoxy-2-oxo-4H-benzo[d][1,3]oxazin-1-yl)-1,1-dimethyl-propylamino]-1-hydroxy-ethyl}-2-hydroxy-phenyl)-methanesulphonamide.

16. Compounds of general formula **1** according to one of claims 13 to 15, wherein the compounds of formula **1** are in the form of the individual optical isomers, mixtures of the individual enantiomers or optionally diastereomers or racemates, preferably in the form of the enantiomerically pure, optionally diastereomerically pure compounds.

17. Compounds of general formula **1** according to one of claims 13 to 15, wherein the compounds of formula **1** are in the form of the free bases or in the form of the acid addition salts with pharmacologically acceptable acids as well as optionally in the form of the solvates and/or hydrates.

18. Compounds of general formula **1** according to one of claims 13 to 17 as medicaments.

19. Pharmaceutical formulation, **characterised in that** it contains a compound of formula **1** according to one of claims 13 to 17.

## Revendications

1. Utilisation de composés de formule générale 1 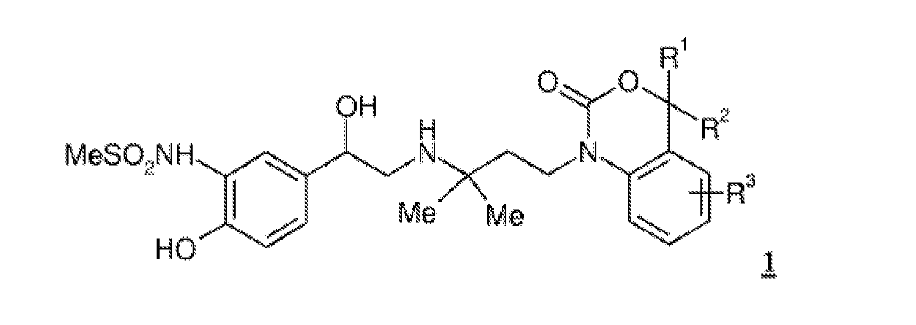 dans laquelle
R¹ et R² identiques ou différents, désignent un atome d'hydrogène, d'halogène, un groupe alkyle en C₁ à C₄ ou conjointement, un groupe alkylène en C₁ à C₆ et
R³ désigne un atome d'hydrogène, d'halogène, OH, un groupe alkyle en C₁ à C₄ ou -O-alkyle en C₁ à C₄ ; pour la production d'un médicament destiné au traitement de maladies des voies respiratoires qui sont choisies dans le groupe comprenant des maladies pulmonaires obstructives d'origines différentes, des emphysèmes pulmonaires d'origines différentes, des maladies pulmonaires restrictives, des maladies pulmonaires interstitielles, des fibroses kystiques, des bronchites d'origines différentes, des bronchiectasies, le SDRA et toutes les formes d'oedèmes pulmonaires.

2. Utilisation de composés de formule générale 1 selon la revendication 1, dans laquelle,
R¹ et R², identiques ou différents, désignent un atome d'hydrogène, de fluor, de chlore, un groupe méthyle, éthyle, propyle, butyle ou conjointement -CH₂-CH₂-, -CH₂-CH₂-CH₂-, -CH₂-CH₂-CH₂-CH₂- ou -CH₂-CH₂-CH₂-CH₂-CH₂- ;
R³ désigne un atome d'hydrogène, de fluor, de chlore, OH, un groupe méthyle, éthyle, méthoxy ou éthoxy.

3. Utilisation de composés de formule générale 1 selon la revendication 1, dans laquelle,
R¹ et R², identiques ou différents, désignent un atome d'hydrogène, un groupe méthyle, éthyle, propyle ou conjointement -CH₂-CH₂-, -CH₂-CH₂-CH₂-, -CH₂-CH₂-CH₂-CH₂- ou -CH₂-CH₂-CH₂-CH₂-CH₂- ;
R³ désigne un atome d'hydrogène, de fluor, OH, un groupe méthyle ou méthoxy.

4. Utilisation de composés de formule générale 1 selon l'une des revendications 1 à 3, pour la production d'un médicament destiné au traitement de maladies pulmonaires obstructives qui sont choisies dans le groupe comprenant l'asthme bronchique, l'asthme pédiatrique, l'asthme sévère, les crises d'asthme aiguës, la bronchite chronique et la BPOC, l'utilisation pour la production d'un médicament pour le traitement de l'asthme bronchique ou pour le traitement de la BPCO étant particulièrement préférée.

5. Utilisation de composés de formule générale 1 selon l'une des revendications 1 à 3, pour la production d'un médicament destinés au traitement des emphysèmes pulmonaires qui ont leur origine dans la BPCO ou dans un défaut de l'inhibiteur de l'α1-protéinase.

6. Utilisation de composés de formule générale 1 selon l'une des revendications 1 à 3, pour la production d'un médicament destiné au traitement des maladies pulmonaires restrictives qui sont choisies dans le groupe comprenant l'alvéolite allergique, des maladies pulmonaires restrictives déclenchées par des substances nocives professionnelles telles que l'asbestose ou la silicose et une restriction due à des tumeurs pulmonaires telles que par exemple, la lymphangiose carcinomateuse, le carcinome broncho-alvéolaire et les lymphomes.

7. Utilisation de composés de formule générale 1, selon l'une des revendications 1 à 3, pour la production d'un médicament destiné au traitement des maladies pulmonaires interstitielles, qui sont choisies dans le groupe comprenant les pneumonies d'origine infectieuse telles que par exemple dues à une infection par des virus, des bactéries, des champignons, des protozoaires, des vers ou autres agents pathogènes, des pneumopathies de différentes origines talles que par exemple, la broncho-aspiration et l'insuffisance cardiaque gauche, les pneumopathies ou fibroses induites par des rayons, les collagénoses telles que par exemple, le lupus érythémateux, la sclérodermie systémique ou la sarcoïdose, les granulomatoses telles que par exemple, la maladie de Boeck, la pneumonie interstitielle idiopathique ou la fibrose pulmonaire idiopathique (FPI).

8. Utilisation de composés de formule générale 1 selon l'une des revendications 1 à 3, pour la production d'un médicament destiné au traitement de fibroses kystiques ou des mucoviscidoses, des bronchiectasies ou le SDRA (syndrome de détresse respiratoire de l'adulte).

9. Utilisation de composés de formule générale 1 selon l'une des revendications 1 à 3, pour la production d'un médicament destiné au traitement des bronchites dues à une infection bactérienne ou virale, une bronchite allergique et une bronchite toxique.

10. Utilisation de composés de formule générale 1 selon l'une des revendications 1 à 3, pour la production d'un médicament destiné au traitement des oedèmes pulmonaires, par exemple, des oedèmes pulmonaires toxiques après aspiration ou inhalation de substances toxiques et de substances étrangères.

11. Utilisation de composés de formule générale 1 selon l'une des revendications 1 à 10, les composés de formule 1 se présentant sous la forme des isomères optiques individuels, de mélanges des énantiomères individuels, éventuellement de diastéréomères ou de racémates, de préférence sous la forme de composés purement énantiomères ou purement diastéréomères.

12. Utilisation de composés de formule générale 1 selon l'une des revendications 1 à 10, les composés de formule 1 se présentant sous la forme de bases libres ou sous la forme de sels d'addition acides avec des acides pharmacologiquement acceptables et éventuellement sous la forme de solvate et/ou d'hydrates.

13. Composés de formule 1 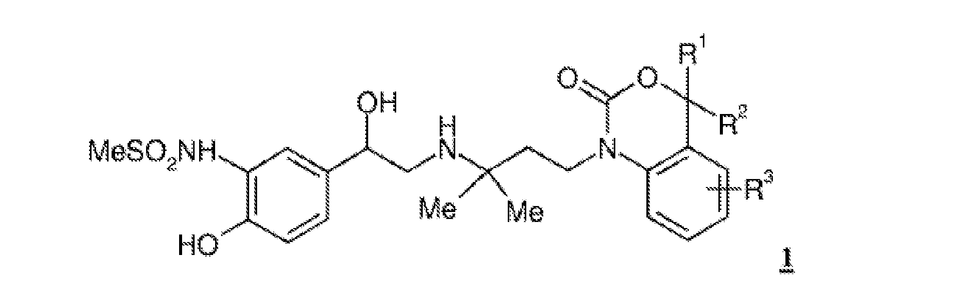 dans laquelle
R¹ et R² identiques ou différents, désignent de préférence -CH₂-CH₂-, -CH₂-CH₂-CH₂-, -CH₂-CH₂-CH₂-CH₂- ou -CH₂-CH₂-CH₂-CH₂-CH₂- et
R³ désigne un atome d'hydrogène, de fluor, de chlore, OH, un groupe méthyle, éthyle, méthoxy ou éthoxy.

14. Composés de formule 1 selon la revendication 13, dans laquelle
R¹ et R², identiques ou différents, désignent de préférence, un groupe éthyle ou propyle, ou conjointement -CH₂-CH₂-, -CH₂-CH₂-CH₂-, -CH₂-CH₂-CH₂-CH₂- ou -CH₂-CH₂-CH₂-CH₂-CH₂- ; et dans laquelle
R³ peut être un atome d'hydrogène, de fluor, OH, un groupe méthyle ou méthoxy, de préférence, un atome d'hydrogène.

15. Composés de formule 1 selon la revendication 13 ou 14, qui sont choisis dans le groupe comprenant :
- le N-(5-{2-[1,1-diméthyl-3-(2-oxo-4,4-dipropoxyl-4H-benzo[d][1,3]oxazin-1-yl)-propylamino]-1-hydroxy-éthyl}-2-hydroxy-phényl)-méthanesulfonamide ;
- le N-[5-(2-{1,1-diméthyl-3-[spiro(cyclohexane-1,4'-2H-3',1'-benzoxazin)-2'-oxo-1-yl]-propylamino}-1-hydroxy-éthyl)-2-hydroxy-phényl]-méthanesulfonamide ;
- le N-[5-(2-{1,1-diméthyl-3-[spiro(cyclopropyl-1,4'-2H-3',1'-benzoxazin)-2'-oxo-1-yl]-propylamino}-1-hydroxy-éthyl)-2-hydroxy-phényl]-méthanesulfonamide ;
- le N-(5-{2-[3-(4,4-diéthyl-2-oxo-4H-benzo[d][1,3]oxazin-1-yl)-1,1-diméthyl-propylamino]-1-hydroxy-éthyl}-2-hydroxy-phényl)-méthanesulfonamide ;
- le N-(5-{2-[3-(4,4-diéthyl-6-fluoro-2-oxo-4H-benzo[d][1,3]oxazin-1-yl)-1,1-diméthyl-propylamino]-1-hydroxy-éthyl}-2-hydroxy-phényl)-méthanesulfonamide ;
- le N-(5-{2-[3-(4,4-diéthyl-7-fluoro-2-oxo-4H-benzo[d][1,3]oxazin-1-yl)-1,1-diméthyl-propylamino]-1-hydroxy-éthyl}-2-hydroxy-phényl)-méthanesulfonamide ;
- le N-(5-{2-[3-(4,4-diéthyl-8-méthoxy-2-oxo-4H-benzo[d][1,3]oxazin-1-yl)-1,1-diméthyl-propylamino]-1-hydroxy-éthyl}-2-hydroxy-phényl)-méthanesulfonamide ;
- le N-(5-{2-[3-(4,4-diéthyl-6-méthoxy-2-oxo-4H-benzo[d][1,3]oxazin-1-yl)-1,1-diméthyl-propylamino]-1-hydroxy-éthyl}-2-hydroxy-phényl)-méthanesulfonamide.

16. Composés de formule générale 1 selon l'une des revendications 13 à 15, les composés de formule 1 se présentant sous la forme des isomères optiques individuels, de mélanges d'énantiomères individuels éventuellement sous forme de diastéréomères ou de racémates, de préférence sous la forme de composés purement énantiomères ou purement diastéréomères.

17. Composés de formule générale 1 selon l'une des revendications 13 à 15, les composés de formule 1 se présentant sous la forme de bases libres ou sous la forme de sels d'addition acides avec des acides pharmacologiquement acceptables et éventuellement sous la forme de solvate et/ou d'hydrates.

18. Composés de formule générale 1 selon l'une des revendications 13 à 17 comme médicament.

19. Formulation pharmaceutique, **caractérisée par** une teneur en composé de formule 1 selon les revendications 13 à 17.
